# EUROPÄISCHE PATENTANMELDUNG

(11) **EP 4 186 469 A1**
(43) Veröffentlichungstag der Anmeldung: **31.05.2023**
(21) Anmeldenummer: 21210523.3
(22) Anmeldetag: 25.11.2021
(51) Int. Cl.: A61C 19/04, A61B 1/24, G06T 7/70, A61B 5/00, A61B 1/00

(54) **PERIODONTALHANDGERÄT**

(71) Anmelder: Ivoclar Vivadent AG, 9494 Schaan (LI)
(72) Erfinder: GROSSE-HONEBRINK, Alexander, 9320 Arbon (CH); NIEDRIG, Christian, 9478 Azmoos (CH); REINHARDT, Jonas, 7206 Igis (CH); KUHN, Marvin, 6800 Feldkirch (AT)
(74) Vertreter: Baldus, Oliver

(57) **Zusammenfassung**

Die vorliegende Erfindung betrifft ein Periodontalhandgerät (100) zum elektronischen Erfassen eines Periodontalstatus, mit einer elektronischen Kamera (105) zum Erfassen eines Bildes einer Periodontalsonde (101) in einer Zahnfleischtasche (103); und einer elektronischen Bildauswertungseinrichtung (123) zum Berechnen einer Eindringtiefe der Periodontalsonde (101) auf Basis des erfassten Bildes.

## Beschreibung

Die vorliegende Erfindung betrifft ein Periodontalhandgerät zum elektronischen Erfassen eines Periodontalstatus, eine Manschette zum Überziehen über ein Periodontalhandgerät, ein Periodontalhandgerätesystem und ein Verfahren zum elektronischen Erfassen eines Periodontalstatus.

Die Erfassung des Periodontalstatus eines Patienten wird heute manuell mittels einer Periodontalsonde durchgeführt, auf der eine Messskala eingraviert ist. Diese Messskala wird von einem Benutzer abgelesen. Das Ergebnis wird anschließend mündlich an eine weitere Person weitergegeben, die dieses manuell in ein Periodontalchart des Patienten einträgt. Dieses Verfahren ist aufwändig und fehleranfällig.

Die Druckschrift EP 3 648 703 A1 beschreibt einen Intraoralscanner zur Erfassung der dreidimensionalen Struktur mit einer Sonde.

Die Druckschrift WO 98/06352 A1 beschreibt die fotografische Aufnahme einer herkömmlichen Periodontalsonde in einer Gingivatasche mit einer separaten Intraoralkamera. Die Verarbeitung der gewonnen Bilder erfolgt auf einem getrennten Computer.

Es ist die technische Aufgabe der vorliegenden Erfindung, eine schnelle und einfache Erfassung eines Periodontalstatus mit einem einzigen Handgerät zu ermöglichen.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Periodontalhandgerät zum elektronischen Erfassen eines Periodontalstatus gelöst, mit einer elektronischen Kamera zum Erfassen eines Bildes einer Periodontalsonde in einer Zahnfleischtasche; und einer elektronischen Bildauswertungseinrichtung zum Berechnen einer Eindringtiefe der Periodontalsonde auf Basis des erfassten Bildes. Für das Berechnen der Eindringtiefe werden die zweidimensionalen Bilder ausgewertet, die durch die elektronische Kamera erfasst worden sind. Durch die Bildauswertungseinrichtung innerhalb des Periodontalhandgeräts wird die schnelle Bestimmung einer Taschentiefe einer Zahnfleischtasche ermöglicht und die Handhabung des Periodontalhandgerät vereinfacht. Das Periodontalhandgerät dient zur automatischen, elektronischen Erfassung des Periodontalstatus, die von einem einzigen Benutzer ausgeführt werden kann. Dabei können die Zahnfleischtaschentiefe und die Zahnfleischgrenze in Relation zum Zahn mittels der Periodontalsonde und der Kamera gemessen werden.

In einer technisch vorteilhaften Ausführungsform des Periodontalhandgeräts umfasst das Periodontalhandgerät eine Periodontalsonde zum Eindringen in die Zahnfleischtasche und/oder eine Temperaturmessvorrichtung zum Messen einer Körpertemperatur. Die Periodontalsonde kann auf das Periodontalhandgerät aufsetzbar sein. Die Periodontalsonde kann einen Maßstab oder ein Muster zum Bestimmen der Eindringtiefe umfassen. Die Temperaturmessvorrichtung kann in der Periodontalsonde intergiert sein. Dadurch wird beispielsweise der technische Vorteil erreicht, dass Entzündungen des Zahnfleisches erkannt werden können.

In einer weiteren technisch vorteilhaften Ausführungsform des Periodontalhandgeräts umfasst das Periodontalhandgerät eine Kraftmessvorrichtung zum Messen einer Kraft auf die Periodontalsonde. Dadurch wird beispielsweise der technische Vorteil erreicht, dass die Taschentiefe der Zahnfleischtasche bei einer bestimmten Kraft bestimmt werden kann.

In einer weiteren technisch vorteilhaften Ausführungsform des Periodontalhandgeräts ist die elektronische Kamera in einer Vertiefung, an einem Vorsprung oder in einer Gehäuseebene angeordnet. Dadurch wird beispielsweise der technische Vorteil erreicht, dass die elektronische Kamera an besonders geeigneten Stellen angeordnet ist.

In einer weiteren technisch vorteilhaften Ausführungsform des Periodontalhandgeräts sind die elektronische Kamera und/oder die Periodontalsonde an der Stirnseite eines Halteelements oder nebeneinander in dem Haltelement angeordnet. Dadurch wird beispielsweise der technische Vorteil erreicht, dass die Handhabung des Periodontalhandgeräts verbessert wird.

In einer weiteren technisch vorteilhaften Ausführungsform des Periodontalhandgeräts ist die elektronische Kamera und/oder die Periodontalsonde drehbar an einem Halteelement gelagert. Dadurch wird beispielsweise der technische Vorteil erreicht, dass das Periodontalhandgerät an die räumlichen Verhältnisse in einer Mundhöhle angepasst werden kann.

In einer weiteren technisch vorteilhaften Ausführungsform des Periodontalhandgeräts umfasst das Periodontalhandgerät eine weitere elektronische Kamera zum Erfassen eines Bildes der Periodontalsonde in einer Zahnfleischtasche. Durch die beiden Kameras kann zusätzlich der Triangulationswinkel bestimmt werden. Dadurch wird beispielsweise der technische Vorteil erreicht, dass dreidimensionale Information mittels eines stereoskopischen Verfahrens gewonnen werden kann.

In einer weiteren technisch vorteilhaften Ausführungsform des Periodontalhandgeräts ist die eine elektronische Kamera auf der einen Seite der Periodontalsonde angeordnet und die andere elektronische Kamera auf der anderen Seite der Periodontalsonde angeordnet. Dadurch wird beispielsweise der technische Vorteil erreicht, dass die Eindringtiefe der Periodontalsonde in die Zahnfleischtasche beidseitig bestimmt werden kann.

In einer weiteren technisch vorteilhaften Ausführungsform des Periodontalhandgeräts umfasst das Periodontalhandgerät eine Vielzahl elektronischer Kameras zum Erfassen eines Bildes einer Periodontalsonde in der Zahnfleischtasche. Dadurch wird beispielsweise der technische Vorteil erreicht, dass die Eindringtiefe der Periodontalsonde von einer Vielzahl an Positionen aus aufgenommen werden kann und sich die Genauigkeit des Bestimmens der Eindringtiefe verbessert.

In einer weiteren technisch vorteilhaften Ausführungsform des Periodontalhandgeräts sind die elektronischen Kameras um die Periodontalsonde herum angeordnet. Dadurch wird beispielsweise der technische Vorteil erreicht, dass die Eindringtiefe der Periodontalsonde um die gesamte Periodontalsonde herum von zumindest einer Kamera aus bestimmt werden kann.

In einer weiteren technisch vorteilhaften Ausführungsform des Periodontalhandgeräts umfasst das Periodontalhandgerät eine elektronische Datenschnittstelle zum Übermitteln der erfassten Daten. Die Datenschnittstelle kann eine drahtgebundene oder eine drahtlose Datenschnittstelle sein. Dadurch wird beispielsweise der technische Vorteil erreicht, dass die Daten weiterverarbeitet werden können und automatisch in einen externen elektronischen Patientendatensatz übernommen werden können.

In einer weiteren technisch vorteilhaften Ausführungsform des Periodontalhandgeräts umfasst das Periodontalhandgerät eine elektronische Anzeige zum Ablesen der Eindringtiefe der Periodontalsonde. Die Anzeige kann beispielsweise eine Flüssigkristallanzeige sein. Dadurch wird beispielsweise der technische Vorteil erreicht, dass die Eindringtiefe von einem Benutzer direkt am Periodontalhandgerät abgelesen werden kann.

Gemäß einem zweiten Aspekt wird die technische Aufgabe durch eine Manschette zum Überziehen über ein Periodontalhandgerät gelöst, die ein Sichtfenster für die elektronische Kamera des Periodontalhandgeräts umfasst. Dadurch wird beispielsweise der technische Vorteil erreicht, dass für jeden Patienten eine neue und sterile Manschette eingesetzt werden kann und sich der Aufwand für die Desinfektion des Periodontalhandgeräts verringert.

In einer weiteren technisch vorteilhaften Ausführungsform der Manschette umfasst die Manschette eine Dentalsonde. Die Dentalsonde ist beispielsweise eine Periodontalsonde. Dadurch wird beispielsweise der technische Vorteil erreicht, dass für jeden Patienten eine neue und sterile Dentalsonde verwendet werden kann.

Gemäß einem dritten Aspekt wird die technische Aufgabe durch ein Periodontalhandgerätesystem mit einem Periodontalhandgerät nach dem ersten Aspekt und einer Manschette nach dem zweiten Aspekt gelöst. Dadurch werden beispielsweise die entsprechenden technischen Vorteile erreicht wie durch das Periodontalhandgerät oder die Manschette.

Gemäß einem vierten Aspekt wird die technische Aufgabe durch Verfahren zum elektronischen Erfassen eines Periodontalstatus mittels eines Periodontalhandgeräts gelöst, mit den Schritten eines Erfassens eines Bildes einer Periodontalsonde in einer Zahnfleischtasche durch eine elektronische Kamera; und eines Berechnens einer Eindringtiefe der Periodontalsonde auf Basis des erfassten Bildes durch eine elektronischen Bildauswertungseinrichtung innerhalb des Periodontalhandgeräts. Durch das Verfahren werden die gleichen technischen Vorteile wie durch das Periodontalhandgerät nach dem ersten Aspekt erreicht.

In einer technisch vorteilhaften Ausführungsform des Verfahrens wird die Eindringtiefe der Periodontalsonde automatisch in einen Patientendatensatz intergiert. Der Austausch der Daten zwischen einem Patientendatensatz und dem Periodontalhandgerät erfolgt über eine Datenschnittstelle. Dadurch wird beispielsweise der technische Vorteil erreicht, dass die Daten automatisch und schnell dokumentiert werden können.

In einer weiteren technisch vorteilhaften Ausführungsform des Verfahrens wird eine Position des Zahns bestimmt, an dem die Periodontalsonde in die Zahnfleischtasche eindringt. Die Position des Zahns kann automatisch auf Basis eines vorgespeicherten Bildes oder Intraoralscans erfasst werden. Die gemessenen Werte können dann automatisch in Verbindung mit der Position des Zahns im Patientendatensatz gespeichert werden. Dadurch wird beispielsweise der technische Vorteil erreicht, dass sich für jeden Zahn die Daten automatisch erfassen und zuordnen lassen.

Ausführungsbeispiele der Erfindung sind in den Zeichnungen dargestellt und werden im Folgenden näher beschrieben.

Es zeigen:
- Fig. 1: eine schematische Ansicht einer Bauform eines Periodontalhandgeräts;
- Fig. 2: die Wirkungsweise des Periodontalhandgeräts;
- Fig. 3: schematische Ansichten unterschiedlicher Anordnungen von elektronischen Kameras innerhalb des Periodontalhandgeräts;
- Fig. 4A - 4E: schematische Ansichten unterschiedlicher Anordnungen von Kameras bezüglich zur Periodontalsonde;
- Fig. 5: mögliche Markierungen auf einer Periodontalsonde;
- Fig. 6: eine schematische Darstellung einer Manschette;
- Fig. 7: einen Periodontalchart eines Patienten; und
- Fig. 8: ein Blockdiagram eines Verfahrens zum elektronischen Erfassen eines Periodontalstatus.

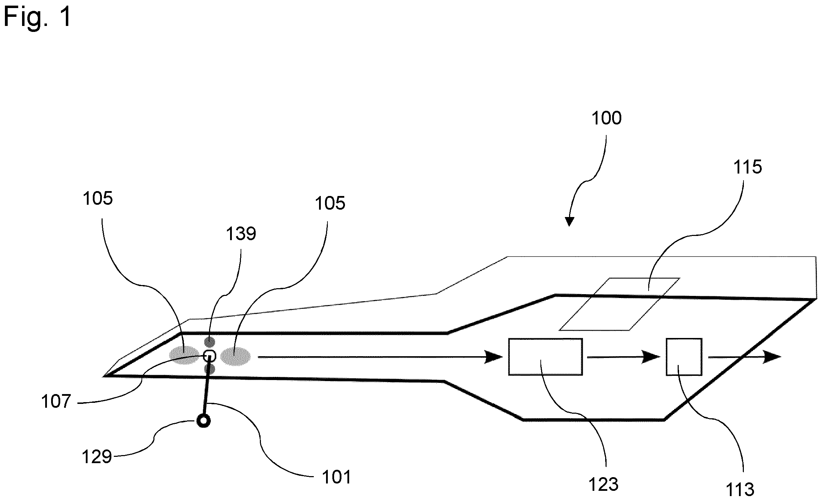

Fig. 1 zeigt eine schematische Ansicht einer Bauform eines Periodontalhandgeräts 100. Das Periodontalhandgerät 100 ist ein tragbares Gerät, das von einem Benutzer während einer Verwendung in einer Hand gehalten werden kann. Elektronische Kameras 105 innerhalb des Periodontalhandgeräts 100 werden so angeordnet, dass diese die Spitze einer Periodontalsonde 101 aufnehmen können. Diese können ebenfalls die Zahnokklusalfläche und die Gingivagrenze aufnehmen. Mittels der Ansichtswinkel kann die Entfernung zwischen der Gingivagrenze und der okklusalen Zahnfläche sowie die Eindringtiefe der Periodontalsonde 101 in die Zahnfleischtasche (Periodontaltasche) berechnet werden. Diese Berechnungen werden von einem Prozessor (CPU) auf Basis der aufgenommenen Bilder berechnet, der in dem Periodontalhandgerät 100 angeordnet ist. Zur Verbesserung der Bildaufnahme umfasst das Periodontalhandgerät 100 eine Lichtquelle 139.

Die okklusale Fläche sowie die Gingivagrenze werden von einer Bilderkennungssoftware erkannt und die Entfernung zwischen diesen unter Zuhilfenahme des Ansichtswinkels des jeweiligen Objekts berechnet. Dadurch können zusätzlich Veränderungen der Gingivagrenze in Relation zum Zahn bei dem jeweiligen Patienten erfasst werden.

Die Taschentiefe der Zahnfleischtasche wird berechnet, wenn die Sonde unter die Gingivagrenze eindringt und eine Druckkraft von 0.2 bis 0.25 N aufgebaut ist. Zu diesem Zweck umfasst das Periodontalhandgerät 100 eine Kraftmessvorrichtung 107 zum Messen einer Kraft auf die Periodontalsonde 101, wie beispielsweise ein elektronisches Newtonmeter. Die Kraftmessung kann in der Periodontalsonde 101 oder in der Aufhängung der Periodontalsonde 101 durchgeführt werden. Die Kraftmessung kann aber auch über die Erkennung der Verformung der Periodontalsonde 101 mittels der Kamera 105 durchgeführt werden. Wird eine vorgegebene Druckkraft auf die Periodontalsonde 101 ausgeübt, wird automatisch ein Bild der Periodontalsonde 101 in einer Zahnfleischtasche 103 aufgenommen. Das Periodontalhandgerät 100 kann aber auch einen Druckknopf umfassen, bei dessen Betätigung eine Aufnahmeauslösung erfolgt.

Die Kraft wird mit einem vorgegebenen Vergleichswert verglichen, der beispielsweise zwischen 0.2 N bis 0.25 N liegt. Das Periodontalhandgerät 100 kann eine Warnvorrichtung umfassen, die ein Warnsignal ausgibt, wenn der vorgegebene Vergleichswert überschritten wird. Mittels Markierungen an der Periodontalsonde 101 wird die Taschentiefe abgelesen, wenn die ausgeübte Kraft einen bestimmten Wert erreicht hat. Optional kann die Periodontalsonde 101 mit einem Temperaturmessvorrichtung 129 ausgestattet sein, wie beispielsweise einem Temperatursensor. Die Temperaturmessvorrichtung 129 misst die Temperatur des Zahnfleisches im Bereich der Periodontalsonde 101, um Entzündungen zu erkennen.

Durch das Periodontalhandgerät 100 wird das Erfassen des Periodontalstatus automatisiert. Der Benutzer kann den Periodontalstatus selbständig ohne weitere personelle Unterstützung erfassen. Dadurch kann der Periodontalstatus mit geringem Zeitaufwand erfasst werden. Zudem macht das Periodontalhandgerät 100 die Aufgabe reproduzierbar, indem eine Kraft und ein Winkel der Periodontalsonde 101 erfasst werden und dem Benutzer eine Rückmeldung geliefert wird. Dies vereinheitlicht den Prozess, auch wenn verschiedene Benutzer dasselbe Protokoll unterschiedlich ausgeführt haben.

Das Periodontalhandgerät 100 umfasst eine Bildauswertungseinrichtung 123 und eine Datenschnittstelle 113. Die Bildauswertungseinrichtung 123 umfasst einen Mikroprozessor und einen digitalen Speicher, in dem Daten und Verarbeitungsprogramme (Software) gespeichert werden können, wie beispielsweise einen RAM-Speicher. Die aufgenommenen Bilder werden von den Kameras 105 in digitaler Form an die Bildauswertungseinrichtung 123 übermittelt und dort in dem digitalen Speicher gespeichert. Die Software der Bildauswertungseinrichtung 123 vergleicht anschließend beispielsweise ein vorgespeichertes digitales Bild der Periodontalsonde 101 mit einem aufgenommenen Bild der Periodontalsonde 101, während diese in der Zahnfleischtasche angeordnet ist. Aus dem flächenmäßigen Unterschied der Periodontalsonde 101 in beiden Bildern lässt sich die Eindringtiefe der Periodontalsonde 101 in die Zahnfleischtasche 103 bestimmen. Eine Messung der Eindringtiefe der Periodontalsonde 101 kann auch anhand einer wie auch immer gearteten Skala auf der Periodontalsonde 101 erfolgen.

Die Datenschnittstelle 113 dient dazu beliebige Daten des Periodontalhandgeräts 100 an ein externes Gerät zu übermitteln und von dem externen Gerät Daten zu empfangen. Die Datenschnittstelle 113 kann beispielsweise eine WLAN- oder Bluetooth-Schnittstelle oder kabelgebundene Schnittstelle sein. Über diese Datenschnittstelle 113 können beispielsweise Daten über die gemessene Taschentiefe an einen externen Computer übermittelt werden, auf dem der Patientendatensatz verarbeitet wird. Der Computer kann die Daten über die gemessene Taschentiefe anschließend in den Patientendatensatz integrieren.

Die Software der Bildauswertungseinrichtung 123 kann die Bilder der elektronischen Kameras 105 somit auswerten und optional automatisch erkennen, an welchem Zahn und an welcher Position die Taschentiefe der Zahnfleischtasche bestimmt wird. Hierzu kann ein Bildabgleich mit einem Intraoralscan erfolgen. Die Zahn- und Positionserkennung kann auch durch eine manuelle Eingabe erfolgen. Dann werden die Daten ausgewertet.

Zusätzlich können klinische Merkdaten errechnet werden, wie der Periodontal Disease Index (PDI) oder der Clinical Attachment Level. Es können von der Software auch automatisch Blutungen oder Eiteraustritte nach der Sondierung erkannt werden und in dem elektronischen Patientendatensatz abgespeichert werden. Zudem kann die Bilderkennung feststellen, dass ein Zahn an einer bestimmten Stelle fehlt. Zudem kann die Software zwischen natürlichen und falschen Zähnen (Krone, Brücke) unterscheiden und diese Daten ebenfalls in den elektronischen Patientendatensatz eintragen.

Im Allgemeinen kann eine Berechnung und Darstellung aller klinisch relevanten Daten in der Software durchgeführt werden. Es können jedoch auch nur Daten ermittelt werden und die Taschentiefe der Zahnfleischtasche wird in einem PC berechnet.

Die Software kann die Daten eines Patienten in den elektronischen Patientendatensatz abspeichern und neue Daten mit bereits bestehenden Daten vergleichen. Auf diese Weise lässt sich der Zustand bei einer Messung und einer zweiten Messung vergleichen. Dem Benutzer zeitliche Veränderungen direkt anzeigt werden, wie beispielsweise eine Wärmeverteilung (Heatmap). Die Software kann ebenfalls eine Trainingsfunktion für den Benutzer umfassen, durch die eine vorgegebene Kraft (0.2 - 0.25 N) gelernt werden kann, die für die Periodontalbehandlung erforderlich ist. Auch der Eindringwinkel der Periodontalsonde 101 kann gelernt werden, wenn mehr als eine Kamera 105 vorhanden ist.

Die von der taktilen Periodontalsonde 101 erfassten Tiefendaten können aufgrund einer stereoskopisch gewonnenen dreidimensionalen Information mit einem vorhandenen hochauflösenden dreidimensionalen Intraoralscan zusammengeführt werden. Die beobachtenden Kameras 105 können auch von der Periodontalsonde 101 getrennt als herkömmliche Intraoralkamera ausgebildet sein.

Das Periodontalhandgerät 100 umfasst eine elektronische Anzeige 115 zum Ablesen der Eindringtiefe der Periodontalsonde. Im Prinzip können alle Daten visuell dargestellt werden und automatisch in den elektronische Patientendatensatz eingehen, so dass der Periodontalstatus eines Patienten zwischen den Messungen automatisch verglichen und auf einfache Weise dargestellt werden kann.

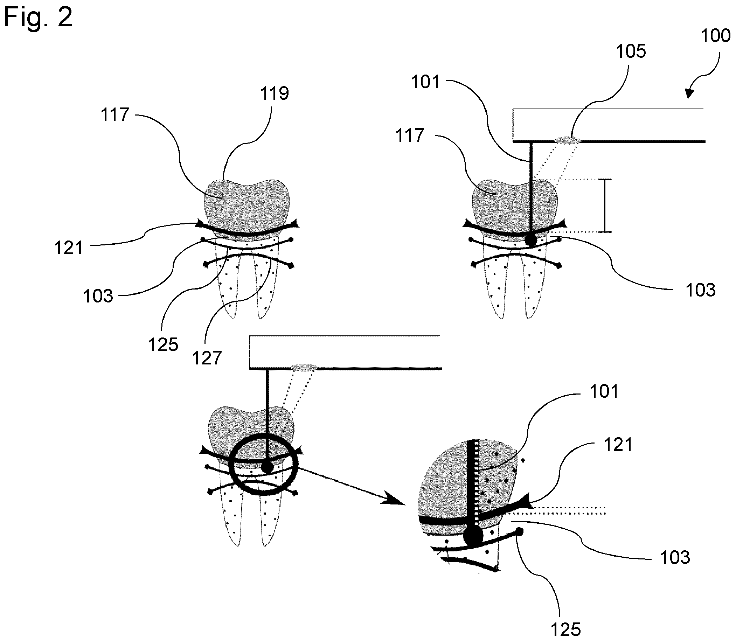

Fig. 2 zeigt die Wirkungsweise des Periodontalhandgeräts 100. Der Zahn 117 umfasst eine Okklusalfläche 119 und eine Gingivagrenze 121. Unterhalb der Gingivagrenze 121 befindet sich zwischen dem Zahn 117 und dem Zahnfleisch (Gingiva) die Zahnfleischtasche 103 mit einer bestimmten Taschentiefe 125. Darunter befindet sich der Alveolarknochen 127.

Zum einen kann durch das Periodontalhandgerät 100 eine Messung der Entfernung zwischen der Gingivagrenze 121 und der Okklusalfläche 119 auf Basis einer Bilderkennung durchgeführt werden. Zum anderen kann eine Skala auf der Periodontalsonde 101 erfasst und automatisch abgelesen werden. Die Periodontalsonde 101 ist aus einem Material gefertigt, das weicher als der Zahnschmelz und das Dentin ist oder das mit einem weichen Material umhüllt ist.

Auf diese Weise kann die Taschentiefe 125 automatisch bestimmt werden. Das Vorderende des Periodontalhandgeräts 100 ist so klein, dass dieses in den Mund des Patienten passt. Das Periodontalhandgerät 100 kann verschiedene Bauformen haben, um den Gegebenheiten im Intraoralraum Rechnung zu tragen. Beispielsweise können verschiedene Anzahlen von elektronischen Kameras 105 montiert werden.

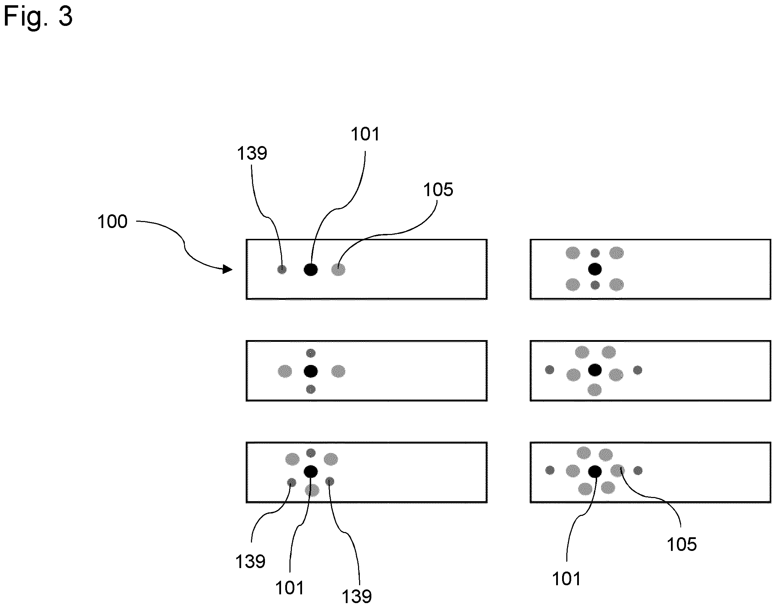

Fig. 3 zeigt unterschiedle Anordnungen von elektronischen Kameras 105 in dem Periodontalhandgerät 100. Das Periodontalhandgerät 100 kann eine oder mehrere elektronischen Kameras 105 umfassen. Die Entfernung der elektronischen Kameras untereinander, der Winkel zueinander und die Entfernung und der Winkel zu der Periodontalsonde 101 sind dabei wählbar und variabel. Das Bild kann dementsprechend korrigiert werden. Der Einlass der elektronischen Kameras 105 in das Gehäuse des Periodontalhandgeräts 100 kann verschieden ausgestaltet sein. Der Einlass der elektronischen Kameras 105 und die Anzahl an Kameras kann kombiniert werden. Zudem kann das Periodontalhandgerät 100 eine oder mehrere Lichtquellen 139 umfassen. Eine Lichtquelle 139 kann auf der einen Seite der Periodontalsonde 101 angeordnet sein und eine andere Lichtquelle 139 kann auf der anderen Seite der Periodontalsonde 101 angeordnet sein. Die Lichtquellen 139 könne auch um die Periodontalsonde 100 herum angeordnet sein.

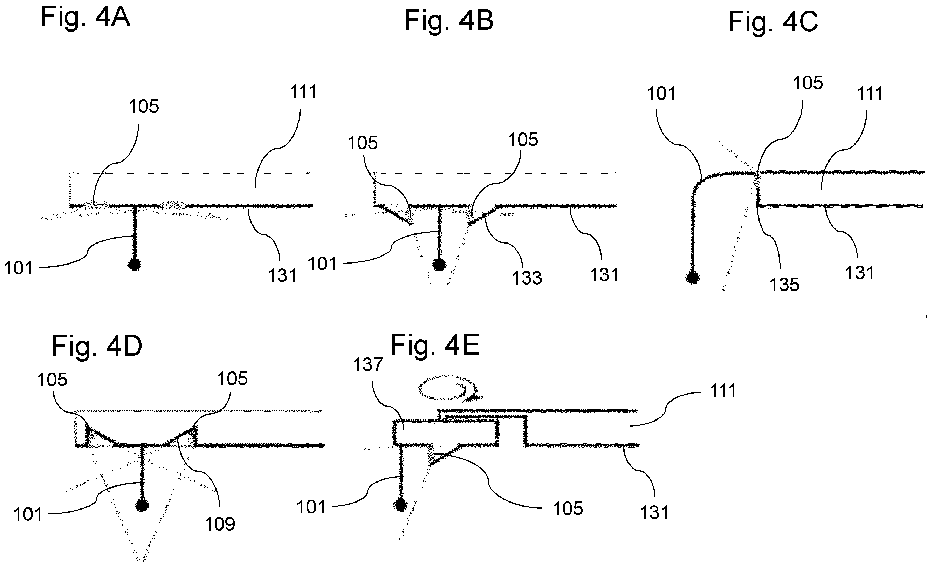

Fig. 4A bis 4E zeigen unterschiedliche Anordnungen der Kamera 105 bezüglich zur Periodontalsonde 101. In Fig. 4A sind die Kameras 105 flach auf oder in das Gehäuse 131 des Haltelements 111 montiert. Hierbei können Weitwinkelkameras als elektronische Kameras 105 verwendet werden. Dadurch wird der technische Vorteil erreicht, dass eine ebene Fläche realisiert wird, die leicht zu reinigen und zu desinfizieren ist.

Fig. 4B zeigt ein Gehäuse 131 mit Vorsprüngen 133, die die Kameras 105 tragen. Die Kameras 105 sind in die Vorsprünge 133 integriert. Dadurch wird der technische Vorteil erreicht, dass der Ansichtswinkel verbessert werden kann.

Fig. 4C zeigt eine Periodontalsonde 101, die frontal an der Stirnseite 135 des Gehäuses 131 des Periodontalhandgeräts 100 montiert ist. Die elektronische Kamera 105 ist ebenfalls an der Stirnseite 135 des Periodontalhandgeräts 100 angeordnet. Dadurch wird der technische Vorteil erreicht, dass auch schwer zugängliche Stellen mit der Periodontalsonde 101 erreicht werden können.

Fig. 4D zeigt elektronische Kameras 105, die in dem Gehäuse 131 des Periodontalhandgeräts 100 unter einem Winkel in Vertiefungen 109 des Haltelements 111 eingelassen sind. Dadurch wird der technische Vorteil erreicht, dass die Optik der Kameras 105 vor Verschmutzungen geschützt ist.

Fig. 4E zeigt ein Periodontalhandgerät 100, bei dem die elektronische Kamera 105 und die Periodontalsonde 101 auf einem Drehteil 137 montiert sind. Das Drehteil 137 ist beispielsweise mit einem Kugelkopfgelenk an einem Haltelement 111 drehbar und kippbar befestigt. Dadurch wird das Messen schwer erreichbarer Stellen erleichtert.

Alle in Fig. 4A bis 4E gezeigten Ausführungsformen können auch kombiniert werden. Zudem kann eine Antibeschlagheizung für die jeweiligen Kameras 105 in dem Periodontalhandgerät 100 vorgesehen sein. Eine Erfassung der Periodontalsonde 101 mit mehreren Kameras 105 erhöht die Genauigkeit der Messung, beispielsweise durch eine Mittelwertbildung der aus jeder Perspektive errechneten Eindringtiefe der Periodontalsonde 101. Bei einer möglichen Verdeckung der Periodontalsonde 101 durch einen anderen Zahn, Speichel oder Blut kann die Eindringtiefe durch Aufnahme aus mehreren Richtungen trotzdem berechnet werden.

Zudem kann eine korrekte Ausrichtung der Periodontalsonde 101 zum Zahn durch eine seitliche Aufnahme aus einem zweiten Blickwinkel überprüft werden. Wenn hierbei erkannt wird, dass die Periodontalsonde 101 parallel zur Zahnseite liegt, ist die Messung der Eindringtiefe und die Bestimmung der Höhe des Zahns zum Zahnfleischrand genau. Außerdem kann eine elastische Verformung oder Verbiegung der Periodontalsonde 101 bei einer Fehlbedienung erkannt werden.

Bei der Verwendung von zwei oder mehreren Kameras 105 kann, vor einer Kamera 105 ein Hochpassfilter positioniert werden, der beispielsweise bei Beleuchtung mit ultraviolettem Licht über eine (grüne) Fluoreszenz eine Unterscheidung zwischen einem natürlichen Zahn und einem künstlichen Zahn ermöglicht.

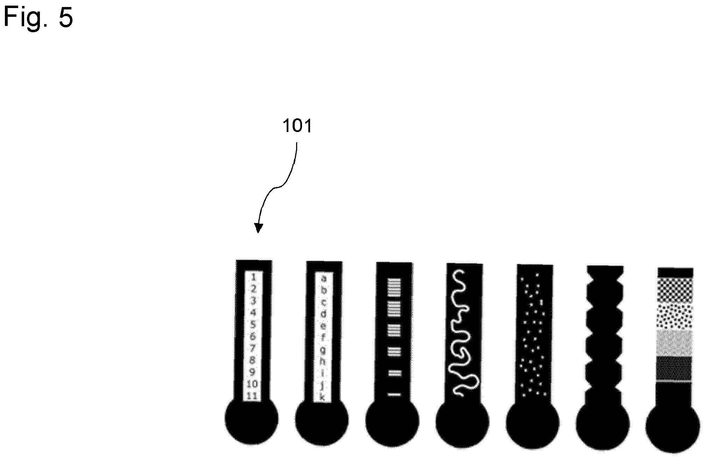

Fig. 5 zeigt mögliche Markierungen auf der Periodontalsonde 101, um die Taschentiefe 125 mittels Bilderkennung abzulesen. Die Periodontalsonde 101 soll von der Kamera 105 erkannt werden, so dass die Eindringtiefe unter die Gingiva ablesbar ist. Zu diesem Zweck können die gezeigten Muster oder Skalen mittels einer Bilderkennung erkannt und abgelesen werden. Bei Skalen kann dies direkt geschehen. Bei Mustern wird dieses erkannt und mit einem vorgespeicherten Muster verglichen. Dadurch kann ein Rückschluss auf die Taschentiefe 125 der Zahnfleischtasche gewonnen werden, welche Teile des Musters noch nicht vom Zahnfleisch verdeckt werden und noch sichtbar sind.

Die Periodontalsonde 101 kann Zahlen oder Buchstaben an verschiedenen Tiefenpositionen, Markierungen wie Striche, römische Zahlen, ein eindeutiges Strich- oder Punktemuster, Einkerbungen in verschiedenen Ausführungen (eckig, rund) oder ein Farb- oder Schattierungsmuster entweder in striktem Verlauf oder in fließenden Übergängen umfassen. Im Allgemeinen sind auch andere Formen sind denkbar.

Weitere Muster können über einen temperaturabhängigen Farbverlauf oder Fluoreszenzverlauf erzeugt werden. Hierdurch können auch Informationen über eine Entzündung des Zahnfleisches erhalten werden. Die Periodontalsonde 101 kann zudem mittels einer Lichtquelle 139 in dem Periodontalhandgerät 100 von außen belichtet werden, wie beispielsweise mit einem Standard-Lichtleiter oder einer Seitenlichtleitfaser (Side Light Fiber), um weitere Daten zu extrahieren. Die Lichtquelle 139 kann neben der elektronischen Kamera 105 angeordnet sein. Die Lichtquelle 139, wie beispielsweise LEDs, kann weißes Licht oder Licht einer spezifischen Wellenlänge aussenden. Wird Licht mit einer geeigneten Wellenlänge verwendet, beispielsweise monochromatisches oder ultraviolettes Licht, lassen sich natürliche und künstliche Zähne voneinander unterscheiden.

Dabei sollte das Muster von der elektronischen Kamera 105 unter einem Winkel abgelesen werden, so dass dieses gut erkennbar ist. Die kleinstmögliche Skala ist daher vom Winkel und der Auflösung der Kamera 105 zur Periodontalsonde 101 abhängig. Des Weiteren kann auch eine Periodontalsonde 101 ohne Skala verwendet werden, wenn mindestens zwei Kameras 105 benutzt werden und der Triangulationswinkel berechnet werden kann. Durch die Triangulation kann eine stereoskopische Erfassung des jeweiligen Zahnes durchgeführt werden, um ein dreidimensionales Modell zu errechnen und dieses mit einem dreidimensionalen Modell aus einem vorhandenen Intraoralscan abzugleichen. Stimmen beide dreidimensionalen Modelle überein, kann der gemessene Werte automatisch dem Zahn in einem Datensatz des Intraoralscans zugeordnet werden.

Aber auch ohne vorhandenen Intraoralscan kann durch die stereoskopische Erfassung ein dreidimensionales Modell errechnet werden, dem die gemessenen Werte zugeordnet sind. Dies ist von Vorteil, wenn die Taschentiefe je Zahn an mehreren Stellen gemessen wird. In diesem können die Positionen auf Basis des dreidimensionalen Modells automatisch erfasst werden.

Allerdings kann die Stereoskopie auch mit nur einer einzigen Kamera 105 durchgeführt werden, wenn softwareseitig ein Vergleich des gerade vermessenen Zahns mit einem zeitlich zuvor aufgenommenen Bild des gesamten Zahnbogens erfolgt. Zu diesem Zweck kann der Benutzer vor Beginn der Messung das Periodontalhandgerät 100 mit den Kameras 105 einmal um den gesamten Zahnbogen führen, um dieses Vergleichsbild aufzunehmen. Des Weiteren kann die Periodontalsonde 101 austauschbar sein, so dass diese separat sterilisierbar ist. Entweder wird nur die Periodontalsonde 101 aus dem Gerät gezogen/geschraubt und ersetzt oder es existiert ein Aufsatz, der auf die Spitze des Periodontalhandgeräts 100 aufgesetzt wird. Der Aufsatz mit der Periodontalsonde 101 kann auch zur Einmalverwendung entwickelt sein. Dies vermindert den Sterilisationsaufwand für den Zahnarzt. Bei einer Verwendung von Periodontalsonden 101 für den Einmalgebrauch (Wegwerfsonden) kann zu Beginn eine Referenzaufnahme der Periodontalsonde 101 durchgeführt werden.

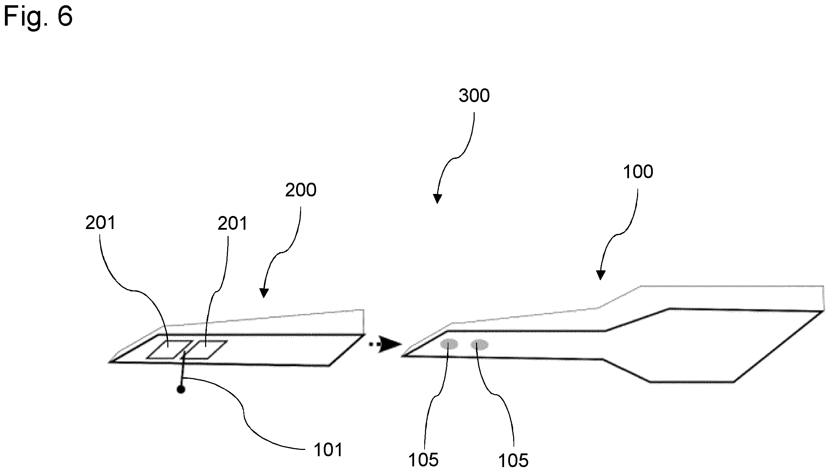

Fig. 6 zeigt eine schematische Darstellung einer Manschette 200. Die Manschette 200 ist sterilisierbar oder ein Einwegartikel und kann über das Periodontalhandgerät 100 gezogen werden. Die Periodontalsonde 101 kann hierbei verschiedene Ausführungen für verschiedene Anwendungen aufweisen, wie beispielsweise zum Periodontalcharting, zur Alveolarknochenbestimmung (Alveolar Bone Sounding) oder zur Furkationsstatusbestimmung. Die Vierecke neben der Periodontalsonde 101 sind Sichtfenster 201, durch die hindurch die Kameras 105 die Periodontalsonde 101 aufnehmen können. Diese Sichtfenster 201 können verschieden ausgestaltet sein, wie beispielsweise durch Glas oder Plastik.

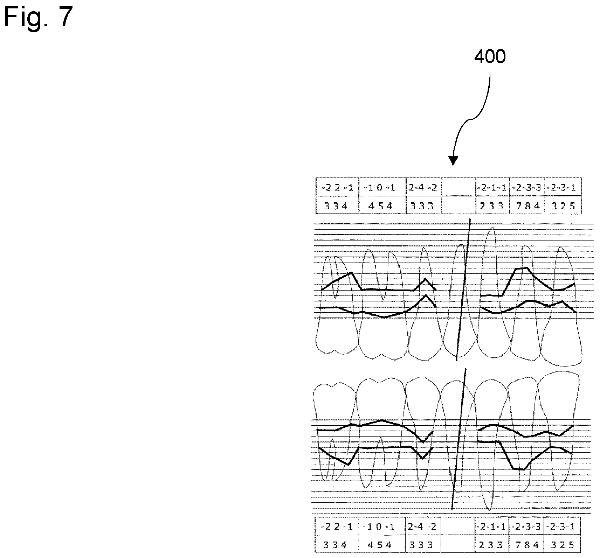

Fig. 7 zeigt einen Periodontalchart 400 eines Patienten. Der Periodontalchart 400 ist als Patientendatensatz gespeichert. In den Periodontalchart 400 werden die gemessenen Werte für die Taschentiefe 125 zu jedem Zahn eingetragen. Zudem kann zu jedem Zahn der Wert für den Abstand zwischen der Okklusalfläche 119 und der Gingivagrenze 121 eingetragen werden. Zu jedem Wert kann das Datum einer Messung gespeichert werden, so dass sich der zeitliche Verlauf erkennen lässt. Auf diese Weise können die Daten der Messungen zur Gingivagrenze 121 und der Taschentiefe in ein elektronisches Periodontalchart 400 des untersuchten Patienten integriert werden.

Dieses Periodontalchart 400 kann auch direkt mit den Bildern der tatsächlichen Zahngegebenheit überlagert werden, d.h. die Bilder werden zu einem 2D-Panorama zusammengenäht (Stitching) und einem vorgegebenen Model überlagert.

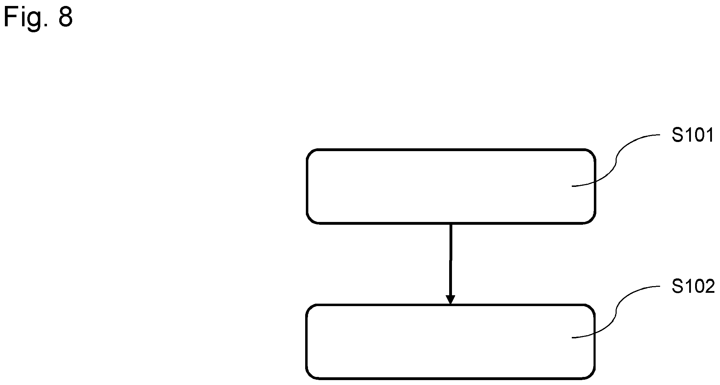

Fig. 8 zeigt ein Blockdiagram eines Verfahrens zum elektronischen Erfassen eines Periodontalstatus mittels des Periodontalhandgeräts 100. Im Schritt S101 wird ein Bild einer Periodontalsonde 101 in einer Zahnfleischtasche 103 durch eine elektronische Kamera 105 erfasst. Anschließend wird in Schritt S102 die Eindringtiefe der Periodontalsonde 101 auf Basis des erfassten Bildes durch die elektronischen Bildauswertungseinrichtung 123 innerhalb des Periodontalhandgeräts 100 erfasst.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLEISTE

- 100: Periodontalhandgerät
- 101: Periodontalsonde
- 103: Zahnfleischtasche
- 105: elektronische Kamera
- 107: Kraftmessvorrichtung
- 109: Vertiefung
- 111: Halteelement
- 113: Datenschnittstelle
- 115: elektronische Anzeige
- 117: Zahn
- 119: Okklusalfläche
- 121: Gingivagrenze
- 123: Bildauswertungseinrichtung
- 125: Taschentiefe
- 127: Alveolarknochen
- 129: Temperaturmessvorrichtung
- 131: Gehäuse
- 133: Vorsprünge
- 135: Stirnseite
- 137: Drehteil
- 139: Lichtquelle
- 200: Manschette
- 201: Sichtfenster
- 300: Periodontalhandgerätesystem
- 400: Periodontalchart

## Patentansprüche

1. Periodontalhandgerät (100) zum elektronischen Erfassen eines Periodontalstatus, mit:
- einer elektronischen Kamera (105) zum Erfassen eines Bildes einer Periodontalsonde (101) in einer Zahnfleischtasche (103); und
- einer elektronischen Bildauswertungseinrichtung (123) zum Berechnen einer Eindringtiefe der Periodontalsonde (101) auf Basis des erfassten Bildes.

2. Periodontalhandgerät (100) nach Anspruch 1, wobei das Periodontalhandgerät (100) eine Periodontalsonde (101) zum Eindringen in die Zahnfleischtasche (103), eine Temperaturmessvorrichtung (129) zum Messen einer Körpertemperatur und/oder eine Kraftmessvorrichtung (107) zum Messen einer Kraft auf die Periodontalsonde (101) umfasst.

3. Periodontalhandgerät (100) nach einem der vorangehenden Ansprüche, wobei die elektronische Kamera (105) in einer Vertiefung (109), an einem Vorsprung (133) und/oder in einer Gehäuseebene angeordnet ist und/oder die elektronische Kamera (105) und/oder die Periodontalsonde (101) an der Stirnseite eines Halteelements (111) angeordnet sind oder nebeneinander in dem Haltelement (111) angeordnet sind.

4. Periodontalhandgerät (100) nach einem der vorangehenden Ansprüche, wobei die elektronische Kamera (105) und/oder die Periodontalsonde (101) drehbar an einem Halteelement (111) gelagert ist.

5. Periodontalhandgerät (100) nach einem der vorangehenden Ansprüche, wobei das Periodontalhandgerät (100) eine weitere elektronische Kamera (105) zum Erfassen eines Bildes der Periodontalsonde (101) in einer Zahnfleischtasche (103) umfasst.

6. Periodontalhandgerät (100) nach Anspruch 5, wobei die eine elektronische Kamera (105) auf der einen Seite der Periodontalsonde (101) angeordnet ist und die andere elektronische Kamera (105) auf der anderen Seite der Periodontalsonde (101) angeordnet ist.

7. Periodontalhandgerät (100) nach einem der vorangehenden Ansprüche, wobei das Periodontalhandgerät (100) eine Vielzahl elektronischer Kameras (105) zum Erfassen eines Bildes einer Periodontalsonde (101) in der Zahnfleischtasche (103) umfasst.

8. Periodontalhandgerät (100) nach Anspruch 7, wobei die elektronischen Kameras (105) um die Periodontalsonde (101) herum angeordnet sind.

9. Periodontalhandgerät (100) nach einem der vorangehenden Ansprüche, wobei das Periodontalhandgerät (100) eine elektronische Datenschnittstelle (113) zum Übermitteln der erfassten Daten umfasst.

10. Periodontalhandgerät (100) nach einem der vorangehenden Ansprüche, wobei das Periodontalhandgerät (100) eine elektronische Anzeige (115) zum Ablesen der Eindringtiefe der Periodontalsonde (101) umfasst.

11. Manschette (200) zum Überziehen über ein Periodontalhandgerät (100), die ein Sichtfenster (201) für die elektronische Kamera (105) des Periodontalhandgeräts umfasst.

12. Manschette (200) nach Anspruch 11, wobei die Manschette (200) eine Dentalsonde (101) umfasst.

13. Periodontalhandgerätesystem (300) mit einem Periodontalhandgerät (100) nach einem der Ansprüche 1 bis 10 und einer Manschette nach einem der Ansprüche 11 oder 12.

14. Verfahren zum elektronischen Erfassen eines Periodontalstatus mittels eines Periodontalhandgerät (100), mit den Schritten:
- Erfassen (S101) eines Bildes einer Periodontalsonde (101) in einer Zahnfleischtasche (103) durch eine elektronische Kamera (105); und
- Berechnen (S102) einer Eindringtiefe der Periodontalsonde (101) auf Basis des erfassten Bildes durch eine elektronischen Bildauswertungseinrichtung (123) innerhalb des Periodontalhandgeräts (100).

15. Verfahren nach Anspruch 14, wobei die Eindringtiefe der Periodontalsonde (101) automatisch in einen Patientendatensatz intergiert wird und/oder eine Position des Zahns bestimmt wird, an dem die Periodontalsonde (101) in die Zahnfleischtasche (103) eindringt.
